# EUROPEAN PATENT APPLICATION

(11) **EP 2 208 738 A1**
(43) Date of publication of application: **21.07.2010**
(21) Application number: 09305022.7
(22) Date of filing: 09.01.2009
(51) Int. Cl.: C07K 14/475, G01N 33/50

(54) **Method for the selection of endothelial cells death inducers via netrin-1 and its applications**

(71) Applicant: Centre National pour la Recherche Scientifique (CNRS), 75016 Paris (FR); Centre Professeur Leon Berard, 69008 Lyon (FR); ENS - Ecole Normale Supérieure de Lyon, 69007 Lyon (FR)
(72) Inventor: Mehlen, Patrick Etienne3 Roger, 69360 Serezin du Rhone (FR); Bernet, Agnès Christine, 69740 Genas (FR); Delloye, Céline Jacqueline Andrée, 69008 Lyon (FR); Castets-Mestrallet, Marie Anne-Sophie Emmanuelle, 69003 Lyon (FR)
(74) Representative: Warcoin, Jacques

(57) **Abstract**

The present invention relates to an *in vitro* method for selecting a compound capable to induce the death of endothelial cells, preferably endothelial cells from vessels or neovessels. The invention further comprises the use of netrin-1 function inhibitors as compounds capable to induce the death of endothelial cells, preferably endothelial cells from vessels or neovessels of tumor expressing netrin-1. Finally, the invention relates to a kit for the selection of a compound capable to induce the death of endothelial cells.

## Description

The present invention relates to an *in vitro* method for selecting a compound capable to induce the death of endothelial cells, preferably endothelial cells from vessels or neovessels. The invention further comprises the use of netrin-1 function inhibitors as compounds capable to induce the death of endothelial cells, preferably endothelial cells from vessels or neovessels of tumor expressing netrin-1. Finally, the invention relates to a kit for the selection of a compound capable to induce the death of endothelial cells.

Netrin-1, originally described as an axon guidance molecule, has recently been shown to function in extra-neural processes as well, ranging from a role in branched organs morphogenesis to a function in angiogenesis (Cirulli and Yebra, 2007; Liu et al., 2004; Lu et al., 2004; Wilson et al., 2006). Interestingly, conflicting results were reported regarding the role of netrin-1 during angiogenesis. However, two clear genetic results initially appeared to be at odds: while Eichmann, Tessier-Lavigne and colleagues elegantly demonstrated that the genetic inactivation of UNC5B in mice is associated with increased angiogenesis, thereby suggesting an anti-angiogenic activity of netrin-1, Li and colleagues showed that the inactivation of netrin-1a, which encodes a ligand for UNC5B, is associated with a loss of vessels during zebrafish development (Lu et al., 2004; Wilson et al., 2006).

Because angiogenesis is an important factor in the initiation and/or proliferation of a large number of diverse disease conditions, there is a need for therapeutical compositions capable of controlling or inhibiting angiogenesis in a reliable manner. Indeed, the process of angiogenesis has been found to be altered in a number of disease states, and in many instances, the pathological damage associated with the disease is related to uncontrolled angiogenesis. For example, angiogenesis is a factor in tumor growth, since a tumor must continuously stimulate growth of new capillary blood vessels in order to grow. Thus, a compound capable to promote or to induce the death of the endothelial cells of blood vessels or neovessels can inhibit the growth of new capillary blood vessels and may be useful for the treatment of these disorders.

It is therefore an object of the invention to provide compounds and pharmaceutical compositions which exhibit activity as inducers of endothelial cells death.

The inventors have demonstrated that the apparent opposite observations which were reported and which led to the contradictory conclusions that netrin-1 is either pro- or anti-angiogenic, result from the fact that netrin-1 acts as a survival factor for endothelial cells, whereas its unbound UNC5B receptor triggers apoptosis of the cells. Netrin-1 effect on blood vessels development is mimicked by caspases inhibitors in ex vivo assays, and inhibition of caspase activity or silencing of a netrin-1 receptor such as UNC5B receptor are both sufficient to rescue the vascular sprouting defects induced by netrin-1 silencing in zebrafish.

Thus, the pro-apoptotic effect of unbound UNC5B and the survival effect of netrin-1-bound UNC5B on endothelial cells probably cooperate during the angiogenesis process.

In a first aspect, the present invention is directed to an *in vitro* method for selecting a compound capable to induce the death of endothelial cells based on the discovery that netrin-1 inhibits endothelial cells apoptosis during angiogenesis.

Thus, the present invention relates to an *in vitro* method for selecting a compound capable to induce the death of endothelial cells wherein said method comprises the following steps of:
a) having a medium containing netrin-1, or a fragment thereof, and endothelial cells;
b) contacting said medium with the compound to be tested;
c) measuring the death of said endothelial cells; and
d) selecting said compound if the measuring in step c) demonstrates a significantly induction of the death of the endothelial cells in presence of said compound.

In a particular embodiment, cell death induced by the presence of the compound to be tested, can be assayed or scored by using the trypan blue staining method as indicated below in the paragraph Material and Methods "cell death assay" (see also Mehlen et al., Nature, 1998).

In another embodiment, the present invention is directed to a method for selecting a compound capable to induce the death of endothelial cells, wherein said method comprises the following steps of:
a) having a medium containing endothelial cells which express at least one netrin-1 receptor;
b) contacting said medium with the compound to be tested;
c) measuring the ability of said compound to be tested to promote the pro-apoptotic activity of this netrin-1 receptor; and
d) selecting said compound as an inducer of the death of endothelial cells, if the measuring in step c) demonstrates that said compound promotes significantly the pro-apoptotic activity of this netrin-1 receptor, preferably in presence further of netrin-1.

In another embodiment, the present invention is directed to a method for selecting a compound capable to induce the death of endothelial cells according to the present invention, wherein:
- the measure in step c) consists to measure the ability of the compound to be tested to induce the activation of the kinase activity of the death-associated protein kinase (DAPK); and

d) selecting said compound as an inducer of the death of endothelial cells, if the measuring in step c) demonstrates a significantly activation of the kinase activity of the death-associated protein kinase (DAPK) in presence of said compound, preferably in presence further of netrin-1.

In another embodiment, the present invention is directed to a method for selecting a compound capable to induce the death of endothelial cells according to claim 2, wherein:
- the measure in step c) consists to measure the ability of the compound to be tested to inhibit the DAPK phosphorylation; and

d) selecting said compound as an inducer of the death of endothelial cells, if the measuring in step c) demonstrates a significantly inhibition of the DAPK phosphorylation in presence of said compound, preferably in presence further of netrin-1.

In another embodiment, the present invention is directed to a method for selecting a compound capable to induce the death of endothelial cells, wherein said method comprises the following steps of:
a) having a medium containing endothelial cells which express at least one netrin-1 receptor and wherein said netrin-1 receptor intracellular domain is able to dimerize or multimerize in presence of netrin-1;
b) contacting said medium with the compound to be tested, the medium further containing netrin-1, or a fragment thereof able to interact with the extracellular domain of the netrin-1 receptor;
c) determining whether the dimerization or multimerization of said netrin-1 receptor intracellular domain is inhibited in presence of said compound to be tested; and
d) selecting said compound as an inducer of the death of endothelial cells, if the determination in step c) demonstrates a significantly inhibition of the dimerization or multimerization of the intracellular domain of said netrin-1 receptor of said endothelial cells.

Determination assay of the inhibition of the dimerization or multimerization of said netrin-1 receptor intracellular domain in presence of a compound to be tested can be found in example in the patent document Mehlen et al., WO 2007/099133 published on September 7, 2007.

In a preferred embodiment, the medium in step a) further contains netrin-1.

In an also preferred embodiment, in the method for selecting a compound capable to induce the death of endothelial cells according to the present invention, said compound is capable to induce the death of the blood vessels or neovessels.

In a preferred embodiment, in step a) of the methods according to the present invention, said endothelial cells are endothelial cells which express at least a netrin-1 receptor selected from the group of DCC, UNC5A, UNC5B, UNC5C and UNC5D, neogenin and the adenosine A2b, preferably UNC5B.

More preferred are endothelial cells which express the netrin-1 receptor UNC5B.

In an also preferred embodiment, in the methods according to the present invention, said endothelial cells in step a) are endothelial cells selected from the group consisting of human or chicken endothelial cells, preferably from human or from chicken vessels or neovessels, more preferably from human umbilical vein endothelial cells (HUVEC) and human arterial endothelial cells (HUAEC) or from chicken chorioallantoic membrane.

In a second aspect, the present invention comprises a compound selected from the group consisting of:
- a compound comprising an extracellular domain of netrin-1 receptor or fragment thereof able to specifically inhibit the interaction between the netrin-1 and said netrin-1 receptor;
- a monoclonal or polyclonal antibody directed specifically against netrin-1 or netrin-1 receptor, particularly directed to the extracellular domain of said netrin-1 receptor or to the netrin-1 fragment able to interact with the extracellular domain of said netrin-1 receptor; and
- a compound capable of inhibiting the netrin-1 expression selected from the group consisting of antisense and iRNA (interfering RNA) oligonucleotides specific of the nucleic acid encoding netrin-1 protein,
as an inducer of endothelial cells death.

Preferably, the invention is directed to such compounds as inducers of endothelial cells death for inducing the death of blood vessels or neovessels.

In a more preferred embodiment, the invention is directed to such compounds as inducers of death of blood vessels or neovessels of tumors.

In an also more preferred embodiment, the tumor cells of said tumor express or overexpress netrin-1.

By the terms interaction between netrin-1 and its netrin-1 receptor in the wording "able to specifically inhibit the interaction between the netrin-1 and said netrin-1 receptor", it is intended to designate in the present application the interaction which result in general from the specific binding between a ligand and its receptor. The inhibition of this interaction can be obtained in general by the complete or partial inhibition of the binding of netrin-1 to its receptor, notably in presence of a competitive ligand (such as an antibody which is directed to this extracellular membrane domain of said netrin-1 receptor), or in presence of a compound able to form a specific complex with the netrin-1 (such as a soluble extracellular membrane domain of its netrin-1 receptor, or part thereof).

Said netrin-1 receptor fragment comprises or is the extracellular domain of the netrin-1 receptor, or part thereof able to specifically interact with netrin-1.

Said netrin-1 or/and said netrin-1 receptor are preferably from mammal, particularly from mouse, rat or human, more preferably from human. Said netrin-1 can be a recombinant netrin-1. Said netrin-1 can be also from chicken.

The term "antibody" as used herein refers to immunoglobulin molecules and immunologically active portions of immunoglobulin molecules, i.e., molecules that contain an antigen binding site which specifically binds (immunoreacts with) the netrin-1 protein or its receptor.

The term "antibody" comprises monoclonal or polyclonal antibodies but also chimeric or humanized antibodies.

An isolated netrin-1 protein or netrin-1 receptor protein, or a specific fragment thereof can be used as an immunogen to generate antibodies that bind such protein using standard techniques for polyclonal and monoclonal antibody preparation. It may be also possible to use any fragment of these proteins which contains at least one antigenic determinant may be used to generate these specific antibodies.

A protein immunogen typically is used to prepare antibodies by immunizing a suitable subject, (e.g., rabbit, goat, mouse or other mammal) with the immunogen. An appropriate immunogenic preparation can contain said protein, or fragment thereof, and further can include an adjuvant, such as Freund's complete or incomplete adjuvant, or similar immunostimulatory agent.

Thus, antibody for use in accordance with the invention include either polyclonal, monoclonal chimeric or humanized antibodies. antibodies able to selectively bind, or which selectively bind to an epitope-containing a polypeptide comprising a contiguous span of at least 8 to 10 amino acids of an amino acid sequence of the netrin-1 protein or its receptor. Intact antibody, or a functional fragment thereof (e.g., Fab or F(ab')2, scFv) can be used.

For example, *in vitro* techniques for detection of candidate oligonucleotides include Northern or Southern hybridizations and in situ hybridizations. *In vitro* techniques for detection of candidate protein include enzyme linked immunosorbent assays (ELISAs), Western blots, immunoprecipitations and immunofluorescence.

The antisense can be an oligonucleotide of at least 10, 15 or 20 nucleotides in length which specifically hybridizes to the mRNA or cDNA, or complementary sequence thereof, of the netrin-1 coding sequence. Preferred are oligonucleotides having at least 95 %, 98 %, 99 % and 100 % identity with the corresponding fragment of the netrin-1 coding sequence.

Interfering RNA (iRNA) is a phenomenon in which a double stranded RNA (dsRNA) specifically suppresses the expression of a gene bearing its complementary sequence. iRNA has since become a useful research tool for many organisms. Although the mechanism by which dsRNA suppresses gene expression is not entirely understood, experimental data provide important insights. This technology has great potential as a tool to study gene function in mammalian cells and may lead to the development of pharmacological agents based upon siRNA (small interfering RNA).

Preferably, in the compounds of the present invention, said extracellular domain of netrin-1 receptor or fragment thereof is selected from the group consisting of DCC, UNC5A, UNC5B, UNC5C and UNC5D, neogenin and the adenosine A2b, more preferably selected from the group of DCC, UNC5A, UNC5B, and UNC5C. UNC5B being the more preferred.

In another aspect, the present invention pertains to a compound according to the present invention, for the preparation of a pharmaceutical composition intended for inhibiting the progression of a cancer for treating a subject suffering having a tumor containing tumoral cells which express or overexpress netrin-1.

Preferably said cancer is selected from the group consisting of breast cancer, colorectal cancer, lung cancer, neuroblastoma, glioma, acute myeloid leukemia, sarcoma, melanoma, ovarian adenocarcinoma, renal adenocarcinoma pancreatic adenocarcinoma, uterus adenocarcinoma, stomac adenocarcinoma, kidney adenocarcinoma and rectal adenocarcinoma.

Preferably said cancer is a metastatic or an aggressive cancer.

When administered to a patient, a compound of the present invention is preferably administered as component of a composition that optionally comprises a pharmaceutically acceptable vehicle. The composition can be administered orally, or by any other convenient route, and may be administered together with another biologically active agent. Administration can be systemic or local. Various delivery systems are known, e.g., encapsulation in liposomes, microparticles, microcapsules, capsules, etc., and can be used to administer the selected compound of the present invention or pharmaceutically acceptable salts thereof.

Methods of administration include but are not limited to intradermal, intramuscular, intraperitoneal, intravenous, subcutaneous, intranasal, epidural, oral, sublingual, intranasal, intracerebral, intravaginal, transdermal, rectally, by inhalation, or topically. The mode of administration is left to the discretion of the practitioner. In most instances, administration will result in the release of the compound into the bloodstream or directly in the primary tumor.

Compositions comprising the compound according to the invention or selected by the methods according to the present invention, form also part of the present invention. These compositions can additionally comprise a suitable amount of a pharmaceutically acceptable vehicle so as to provide the form for proper administration to the patient. The term "pharmaceutically acceptable" means approved by a regulatory agency or listed by a national or a recognized pharmacopeia for use in animals, mammals, and more particularly in humans. The term "vehicle" refers to a diluent, adjuvant, excipient, or carrier with which a compound of the invention is administered. Such pharmaceutical vehicles can be liquids, such as water and oils, including those of petroleum, animal, vegetable or synthetic origin, such as peanut oil, soybean oil, mineral oil, sesame oil and the like. The pharmaceutical vehicles can be saline, gelatin, starch and the like. In addition, auxiliary, stabilizing, thickening, lubricating and coloring agents may be used. Saline solutions and aqueous dextrose and glycerol solutions can also be employed as liquid vehicles, particularly for injectable solutions. Suitable pharmaceutical vehicles also include excipients such as starch, glucose, lactose, sucrose, gelatin, sodium stearate, glycerol monostearate, sodium chloride, dried skim milk, glycerol, propylene, glycol, water and the like. Test compound compositions, if desired, can also contain minor amounts of wetting or emulsifying agents, or pH buffering agents. The compositions of the invention can take the form of solutions, suspensions, emulsion, tablets, pills, pellets, capsules, capsules containing liquids, powders, sustained-release formulations, suppositories, emulsions, aerosols, sprays, suspensions, or any other form suitable for use. Said composition is generally formulated in accordance with routine procedures as a pharmaceutical composition adapted to human beings for oral administration or for intravenous administration. The amount of the active compound or that will be effective in the treatment can be determined by standard clinical techniques. In addition, in vitro or in vivo assays may optionally be employed to help identify optimal dosage ranges. The precise dose to be employed will also depend on the route of administration, and the seriousness of the disease, and should be decided according to the judgment of the practitioner and each patient's circumstances. However, suitable dosage ranges for oral, intranasal, intradermal or intraveneous administration are generally about 0.01 milligram to about 75 milligrams per kilogram body weight per day, more preferably about 0.5 milligram to 5 milligrams per kilogram body weight per day.

In a third aspect, the present invention is directed to a kit for the selection of a compound capable to induce the death of endothelial cells, preferably endothelial cells from vessels or neovessels, wherein said kit comprises:
- a netrin-1 protein or a fragment thereof able to specifically interact with netrin-1 receptor protein to form a binding pair; and
- endothelial cells which express a netrin-1 receptor, preferably selected from the group consisting of DCC, UNC5A, UNC5B, UNC5C and UNC5D, neogenin and the adenosine A2b, more preferably the receptor UNC5B.

In a preferred embodiment, said kit comprises as endothelial cells HUVEC or HUAEC cells.

It is to be understood that while the invention has been described in conjunction with the above embodiments, that the foregoing description and the following examples are intended to illustrate and not limit the scope of the invention. Other aspects, advantages and modifications within the scope of the invention will be apparent to those skilled in the art to which the invention pertains.

### Figures Legends

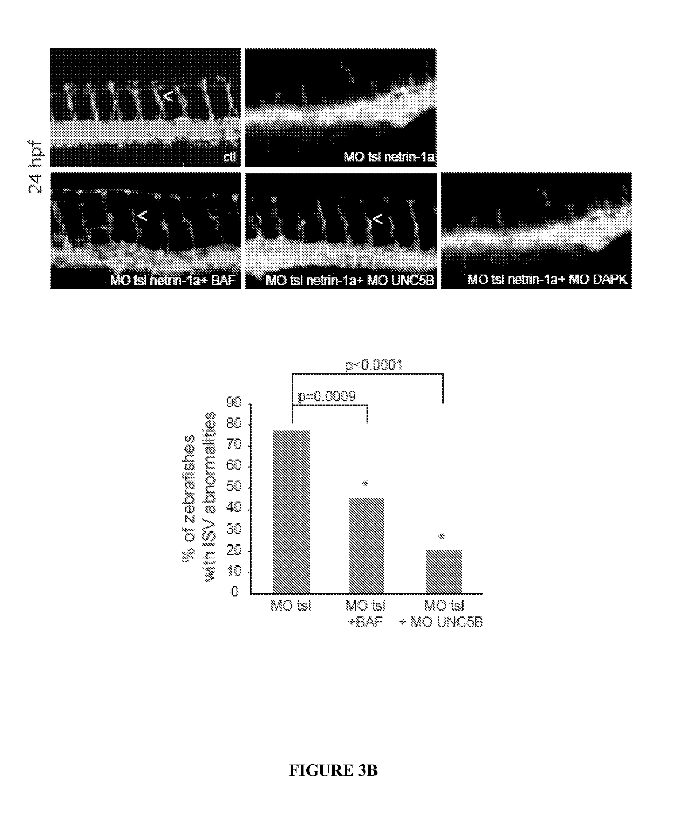

**Figures 1A-1H****:** Netrin-1 prevents endothelial cell apoptosis probably via inhibition of UNC5B-induced apoptosis.
   Figure 1A. Human umbilical vein/artery endothelial cells (HUVEC/HUAEC) express UNC5A and UNC5B dependence receptors, but not netrin-1. Quantitative RT-PCRs were performed as described in Experimental Procedures.
   Figure 1B. Netrin-1 treatment inhibits HUVEC/HUAEC cell death observed upon serum starvation, as measured by trypan blue exclusion. At least one hundred cells per condition were counted. Relative index is shown as the mean +/- s.e.m (n=3). *: p-value<0.005; **: p-value<0.0001 (Student t-test).
   Figure 1C. Netrin-1 prevents apoptosis in HUVEC, as measured by TUNEL staining. Quantification of TUNEL positive cells is mean +/- s.e.m (Student t-test).
   Figures 1D-1E. Netrin-1 treatment reduces caspase-3 activity in HUVEC and HUAEC. Relative index of caspase-3 activity is expressed as the mean +/- s.e.m (n=3). Immunostaining and relative index (mean +/- s.e.m) of cleaved caspase-3 positive cells are shown (Student t-test).
   Figure 1F. UNC5B but not UNC5A silencing by siRNA is associated with a decrease in caspase-3 activity. Values are means and s.e.m (n=3). All p<0.05 (Student t-test compared to level in control condition).
   Figure 1G. DAP kinase mediates UNC5B-induced apoptosis in HUVEC and HUAEC. Cell death (Toxilight) in endothelial cells after forced expression of DAP kinase ΔCaM (constitutively active mutant form of DAP kinase), and of DAP kinase DD (dominant negative form of DAP kinase). Values are mean and s.e.m. All p<0.001 (Student t-test compared to level in control condition).
   Figure 1H. Netrin-1 treatment induces DAP kinase phosphorylation. Anti-phospho-DAP kinase was used to determine level of enzyme activity.
**Figures 2A-2F**: Caspases inhibitors and UNC5B silencing mimic netrin-1 effect on neovessels formation.

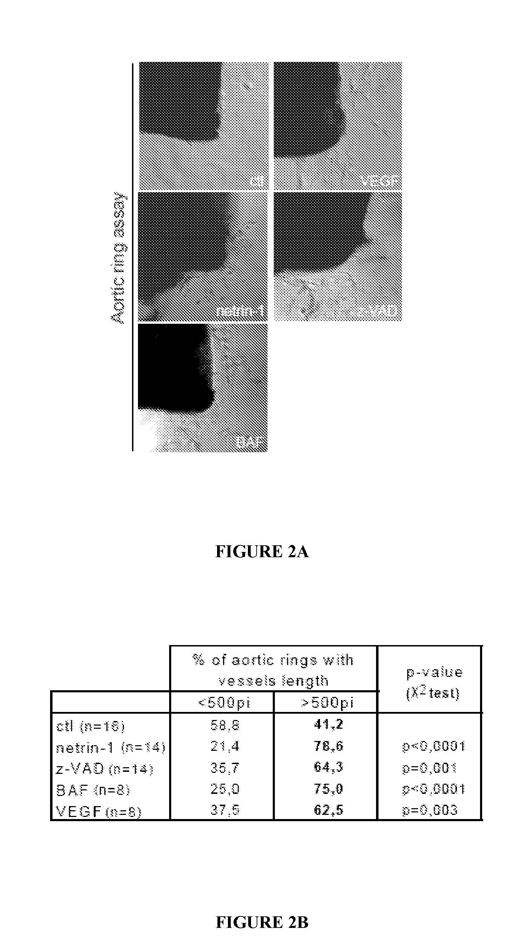

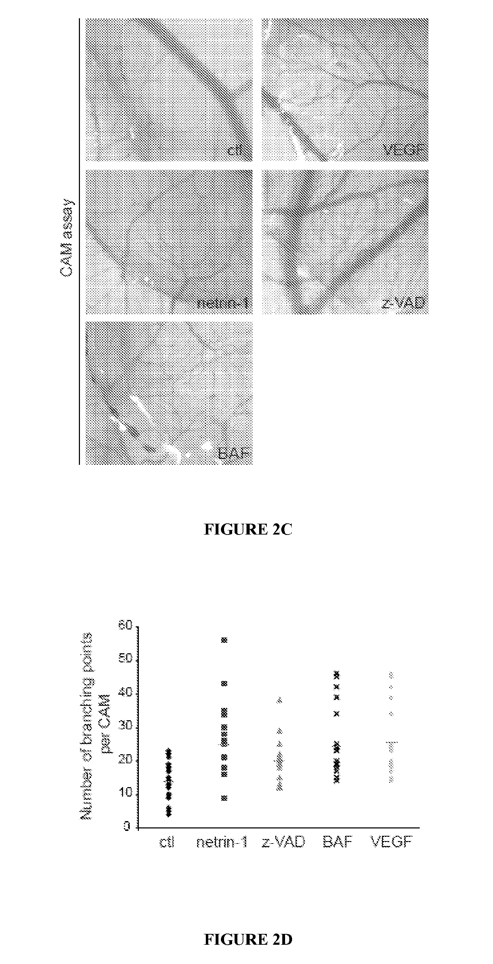

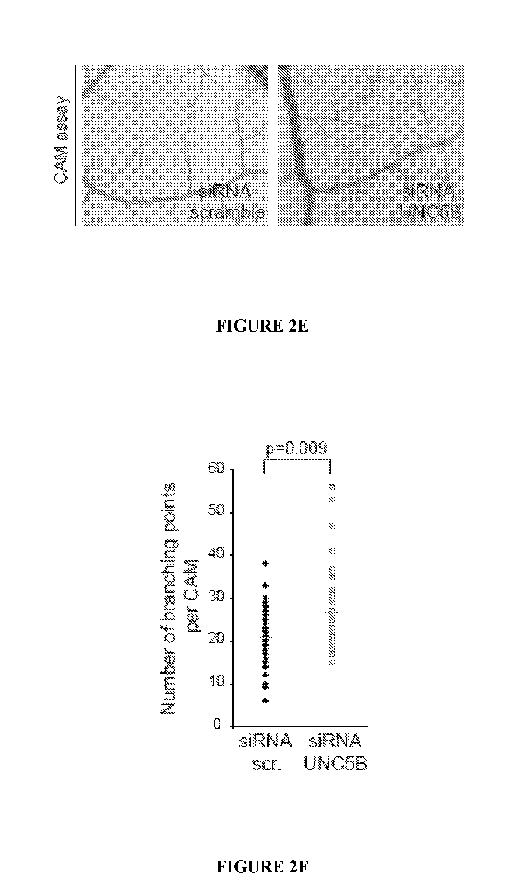

   Figures 2A-2B. Netrin-1 or caspases inhibitors z-VAD-fmk and BAF promote microvessels formation in ex vivo murine aortic ring matrigel assay. Aortic rings resected from at least 8 different mice are shown per condition. Quantification of microvessels total length (pixels units) was performed as described in Experimental Procedures. All p-values are indicated (χ2 test).
   Figures 2C-2D. Netrin-1 and caspases inhibitors induce neovessels formation in CAM assay. CAM models were prepared using 8-day-old chick embryos treated as described in Materials and Methods (n≥10). Methylcellulose disks are outlined. De novo angiogenesis is expressed as the number of branching points (n≥13 fields per condition). Means are indicated by straight lines. *: p-value<0.02; **: p-value<0.0005 (Mann-Whitney U-test).
   Figures 2E-2F. UNC5B silencing by siRNA leads to increase in neovessels formation in CAM assay. Three siRNA injections were performed between E.5 and E.9, as described in Experimental Procedures. Scramble siRNA was used as a control. De novo angiogenesis is expressed as the number of branching points (n>30 different fields per condition). Means are indicated by straight lines. P-value=0.009 (Mann-Whitney U-test).

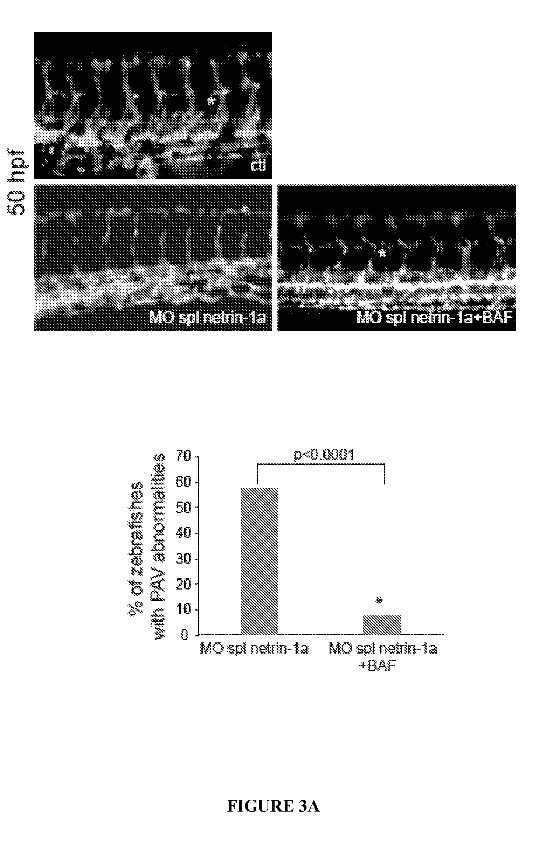

**Figures 3A-3C**: Phenotype of knockdown netrin-1a zebrafish embryos is rescued by inhibition of apoptosis.
   fli:egfp zebrafish embryos were injected at one to four cells stage with control (ctl), splice (spl) or translation (tsl)-blocking netrin1a, UNC5B and DAP kinase morpholinos, alone or in combination. Phenotypes were analysed from 24 to 54 hpf. Representative images of trunk vasculature are shown. Anterior to the left. Caspases inhibitor BAF restores parachordal vessel formation (asterisks) in zebrafish embryos injected with netrin-1a spl morpholinos (Figure 3A) and ISVs formation, in zebrafish embryos injected with netrin-1a tsl morpholinos (Figure 3B). Similar phenotypical rescue was observed by simultaneous injection of UNC5B or DAP kinase and netrin1a tsl-blocking morpholinos in zebrafish embryos (Figure 3B).
   Figures 3A and 3B: quantification is presented. p-values are indicated (χ2 test).

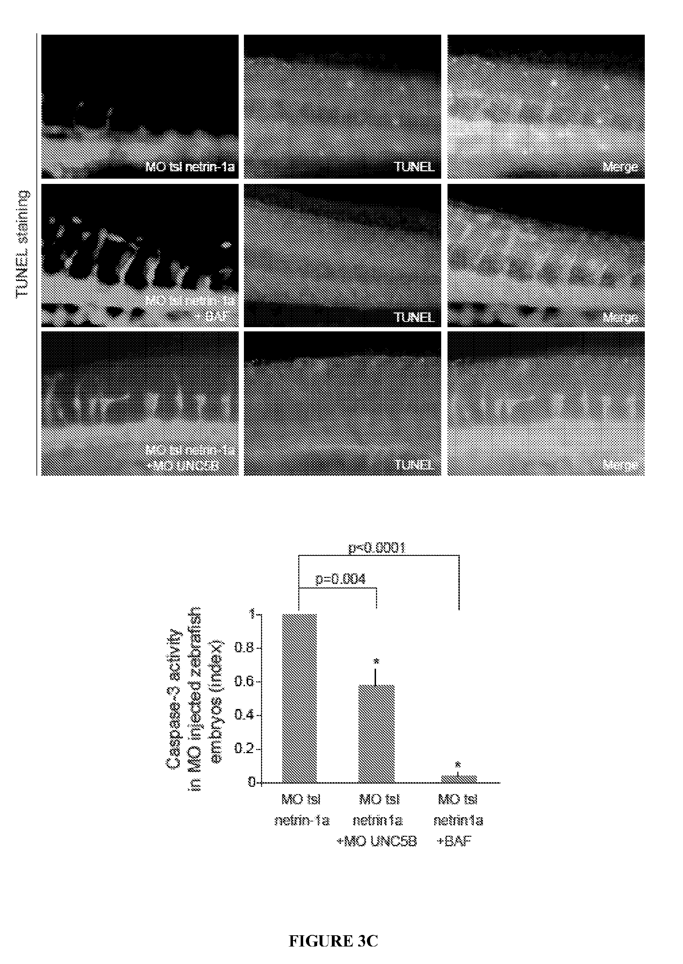

   Figure 3C: BAF treatment or UNC5B-blocking morpholinos injection inhibit the increase in apoptosis observed after netrinla tsl-blocking morpholinos injection in zebrafish embryos, as shown by TUNEL staining (left panels) or caspase-3 activity (right panel). Caspase-3 activity is expressed as a relative index referred to embryos injected with netrin-1a tsl morpholinos solely. Means and s.e.m. are calculated from three independent experiments. p-values are indicated (Student t-test).

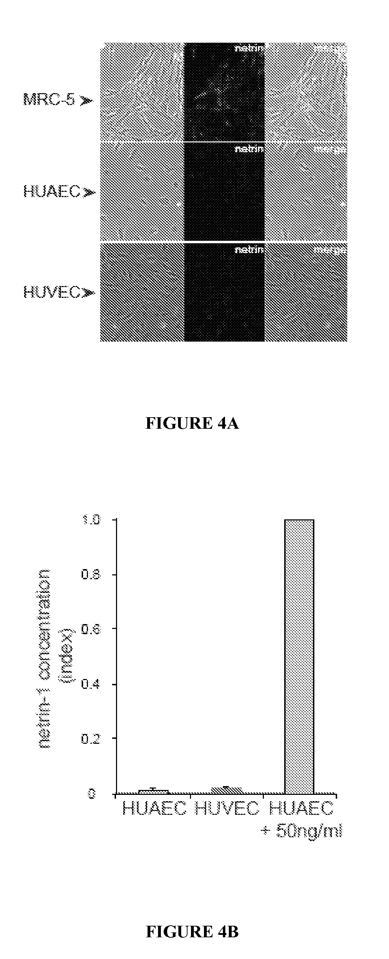

**Figures 4A-4G**: Netrin-1 prevents UNC5B-induced endothelial cell apoptosis.
   Figures 4A-4B. Evaluation of netrin-1 expression in HUVEC and HUAEC cells. Figure 4A. Immunofluorescence using a specific netrin-1 antibody was used. No netrin-1 labeling was detected in HUVEC and HUAEC cells, contrary to MRC-5 lung fibroblast cell line. Figure 4B. Quantitation of netrin-1 secreted in culture medium was performed using Netrin-1 (human) ELISA Kit from Apotech Corporation.

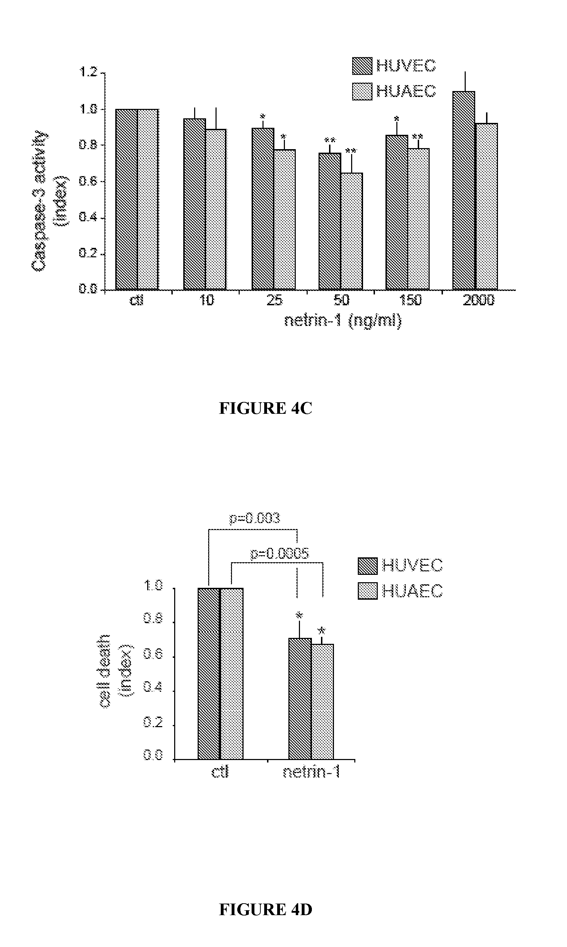

   Figure 4C. Netrin-1 treatment reduces caspase-3 activity in a dose-dependent manner, in HUVEC and HUAEC. Relative index of caspase-3 activity is expressed as the mean +/- s.e.m (n=3). *: p<0.05; **p<0.0025 (Student t-test compared to level in control condition).
   Figure 4D. Netrin-1 treatment inhibits HUVEC/HUAEC cell death observed upon overnight treatment with 50 µM etoposide. Cell death is assessed using Toxilight assay as described in Experimental Procedures. Relative index are means and s.e.m (n=3) (p<0.05, Student t-test).

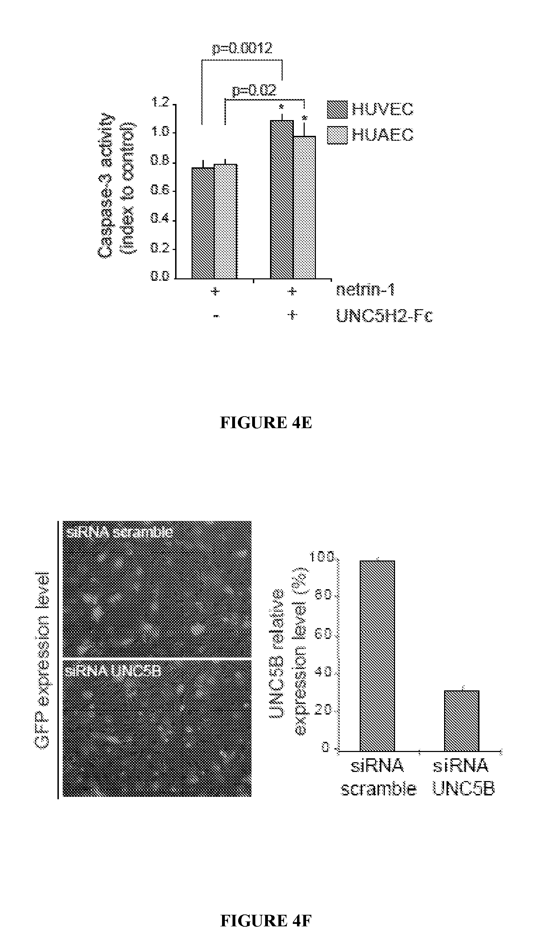

   Figure 4E. Addition of a recombinant protein corresponding to the extracellular domain of rat UNC5H2 prevents netrin-1-mediated cell death inhibition. Results are presented as relative index to controls in each case (means+/- s.e.m, n=3, t-test).
   Figure 4F. Quantitative RT-PCR analysis of UNC5B expression in UNC5B siRNA transfected cells compared to control ones transfected with scramble siRNA. Representative images of GFP expression in siRNA transfected cells, showing efficiency of transfection.

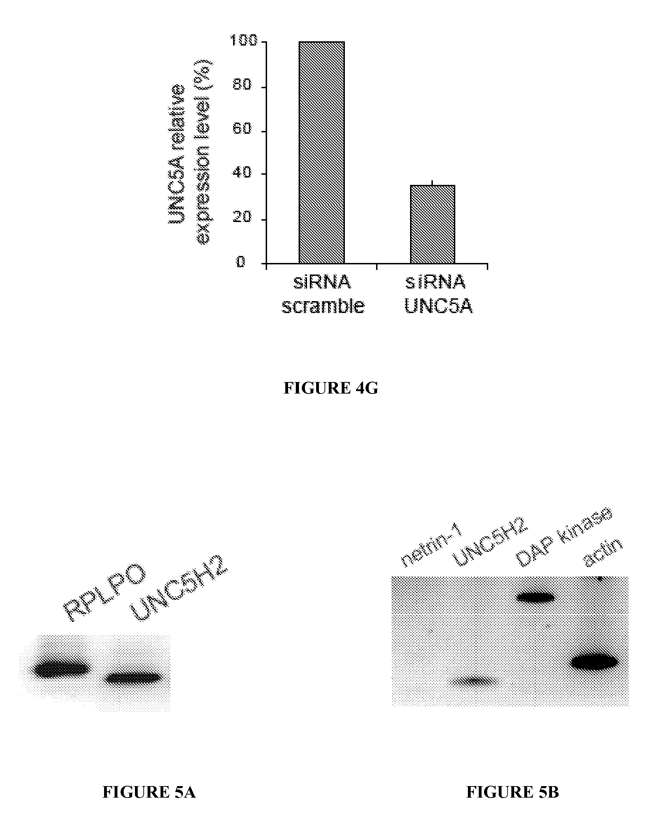

   Figure 4G. Quantitative RT-PCR analysis of UNC5A expression in UNC5A siRNA transfected cells compared to control ones transfected with scramble siRNA.
**Figures 5A-5C****:** Expression of UNC5B in *ex vivo* settings.
   Figure 5A. Quantitative RT-PCR analysis of UNC5B expression in aortic rings.
   Figure 5B. Analysis of netrin-1, UNC5H2 (mouse UNC5B) and DAP kinase expression by quantitative RT-PCR in CAM. Contrary to netrin-1, UNC5H2 and its downstream effector are expressed.

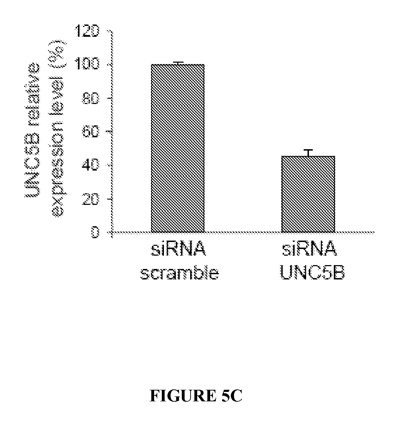

   Figure 5C. Quantitative RT-PCR analysis of UNC5B expression in CAM and effect of UNC5B siRNA injected in blood circulation on UNC5B expression in CAM compared to control ones transfected with scramble siRNA.

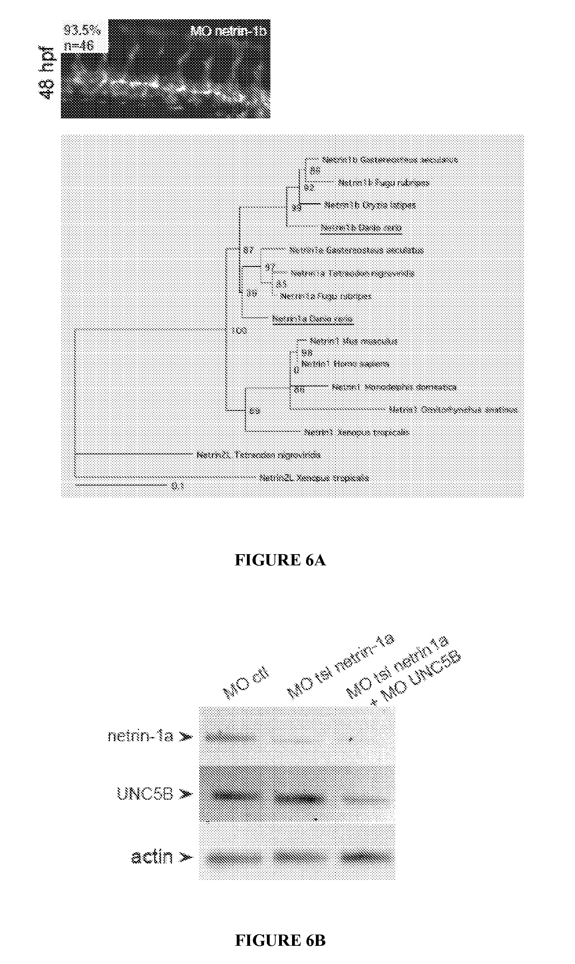

**Figures 6A-6D**: Effect of netrin-1a and UNC5B morpholinos in zebrafish embryos.
   Figure 6A. Netrin-1b does not seem to be involved in developmental angiogenesis in zebrafish. Translation-blocking netrin-1b morpholino has no significant defect on developmental angiogenesis. Percentage of embryos without defects and total number of embryos analysed are indicated. Phylogenic study of netrin-1 otholog genes. ML on protein sequences, under JTT model, with 100 bootstrap replicates. Netrin-1 orthologs in zebrafish, netrin-1a and netrin-1b are underlined.
   Figure 6B. RT-PCR analysis of netrin-1a and UNC5B expression in zebrafish embryos.

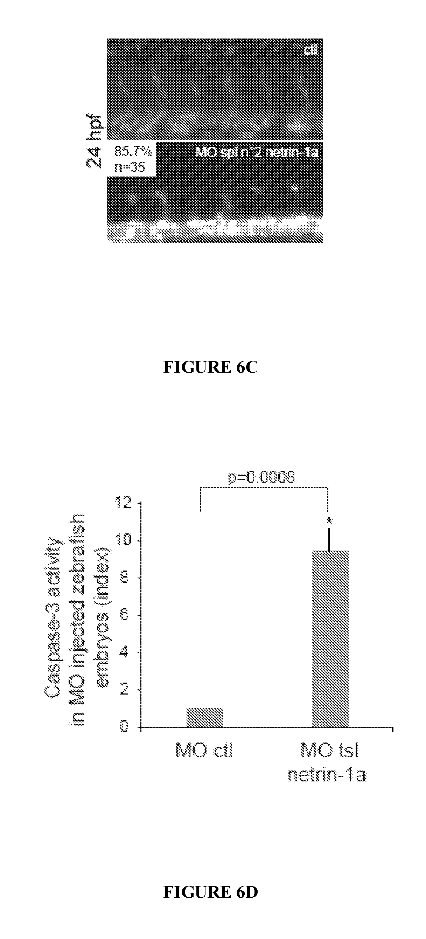

   Figure 6C. Third netrin-1a blocking morpholinos lead to similar vascular defects than both described in Figures 3A-3C. Percentage of embryos with ISVs defects and total number of embryos analysed are indicated.
   Figure 6D. Netrin-1a silencing leads to increase caspase-3 activity in whole zebrafish embryos. Caspase-3 activity is expressed as a relative index referred to embryos injected with control MO. Means and s.e.m. are calculated from three independent experiments.

### Material and Methods

### A) Endothelial cells culture and transfection

### Endothelial cell culture and transfection:

Human umbilical vein and artery cells (Promocell, passages<8) were cultured in endothelial cell growth medium (ECGM, Promocell) supplemented with 2 % serum (kit, Promocell) until they reach 80 % confluence. HUVEC were transfected using the AMAXA Nucleofector system, according to manufacturer's instructions. In brief, 500 000 cells were resuspended in 100 µl HUVEC Nucleofector solution and transfected with 1 µg siRNA or 1.5 µg plasmid DNA and 0.5 µg GFP-encoding control plasmid. UNC5A, UNC5B and scramble siRNAs were designed by Sigma-Proligo. Plasmids encoding mutant DAP kinase (DAP kinase ΔCam and DAP kinase DD) were described previously (Llambi et al., 2005). After transfection, cells were cultured in 1 % growth medium and collected after 24 hours.

### B) Cell death assays

Cell death assays were performed as described previously. Briefly, endothelial cells were seeded at 10 000 cells/cm2 in complete medium for 16 hours. Cell death was induced either by starvation in serum free medium (Endothelium Cell Basal Medium, Promocell) or by exposure to 50 µM etoposide. Netrin-1 was purchased from Axxora-Apotech. Cell death was scored by trypan blue exclusion assay as previously described. For detection of DNA fragmentation, treated cells cultured on coverslips were fixed 20 minutes in 4 % paraformaldehyde (PFA) and Terminal deoxynucleodityl transferase mediated dUTP-biotin Nick End Labeling (TUNEL) was performed with 300 U/mL TUNEL enzyme (300 U/mL) and 6 µM biotinylated dUTP (Roche Diagnostics). Caspase-3 activity was measured using ApoAlert CPP32 kit from Clontech (USA) and by immunostaining on fixed cells using anti-active-caspase-3 antibody (Cell Signaling) as described in (Llambi et al., 2005; Tauszig-Delamasure et al., 2007). Toxilight assay was performed according to manufacturer's instructions (Lonza). For netrin-1 titration experiment, rat recombinant UNC5H2-Fc (R&D) was used at 1 µg/ml.

### C) Western blot analysis and immunofluorescence

Endothelial cells seeded at 10 000 cells/cm² and treated or not with netrin-1 in serum free medium (Endothelium Cell Basal Medium, Promocell) for 24 hours were collected and lysed in 50 mM HEPES pH 7.6, 125 mM NaCl, 5 mM EDTA and 0.1 % NP-40 buffer in the presence of protease inhibitors. Protein extracts were immunoblotted with anti-DAP-kinase and anti-phospho-DAP kinase antibodies (Sigma). For immunofluorescence analysis, cells were seeded on LabTek chamber slides and fixed 15 minutes in 4 % PFA. Anti-netrin-1 rat monoclonal antibody (R&D) was used at 1/150 dilution and revealed using specific secondary antibody (Alexa488 Donkey anti-Rat, Molecular Probes-Invitrogen).

### D) Quantitative RT-PCR

To assay netrin-1, dependence receptors, DCC and UNC5A-D, and DAP kinase expression in HUVEC/HUAEC, aortic rings, CAM and zebrafish embryos, total RNA were extracted using the Nucleospin RNAII kit (Macherey-Nagel) and 1 µg was reverse-transcribed using the iScript cDNA Synthesis kit (BioRad). Real-time quantitative RT-PCR was performed on a LightCycler 2.0 apparatus (Roche) using the Light Cycler FastStart DNA Master SYBERGreen I kit (Roche). Reaction conditions for all optimal amplification, as well as primers selection were determined as already described. The sequences of the primers are available upon request.

### E) Aortic ring assay

Aortic ring assays were performed as described by Nicosia and colleagues (Nicosia and Ottinetti, 1990). Aortas were resected from Balb/C mice of 6 to 8 weeks of age, cleaned of periadventitial fat and connective tissues, rinsed twice in ECGM and cut in rings of 1 to 1.5 mm length. One hundred microliters of Growth factor reduced Matrigel (BD Biosciences) was added to each well of a 48-well tissue culture plate (Costar) and allowed to solidify for 30 min. at 37°C. Aortic rings were placed on the matrix layer, embedded in 100 µl of Matrigel, and allowed to recover in complete growth medium (ECGM, Promocell) for 24 hours. They were then treated or not with netrin-1 (150 ng/ml), z-VAD-fmk (Tebu; 20nM), BAF (Sigma; 20 nM) or VEGF (Sigma; 13 ng/ml) and photographed with a phase contrast microscope on day 4. Microvessels outgrowth total length was calculated using AxioVision Release 4.6 software.

### F) CAM assay

Chick chorioallantoic membrane assay (CAM) assay was performed as already described. White Leghorn eggs were incubated at 37 °C in a humidified atmosphere. After 72 hours of incubation, 3ml albumen was removed and a square window was opened into the shell. The opening was closed with cellotape until day 8. Netrin-1 (150 ng/ml), z-VAD-fmk (Tebu; 20 nM), BAF (Sigma; 20 nM), or VEGF (Sigma) were then diluted in PBS and mixed with an equal volume of autoclaved 1 % methylcellulose solution. Controls were prepared with vehicles only. Each sample solution (10 µl) was dropped on Parafilm and dried up. The methylcellulose disks were stripped off from the Parafilm and placed on a CAM of the 8-day-old chick embryos. After 3 days, CAMs were photographed and the angiogenic response was evaluated by measuring the number of secondary ectopic vessels sprouts under the methylcellulose disks using AxioVision Release 4.6 software. UNC5B silencing in CAM was achieved by injection of specific siRNA designed by Sigma-Proligo in CAM blood circulation at E.5, E.7 and E.9. Analysis was performed at E.11.

### G) Morpholinos knockdown of zebrafish embryos

Transgenic fli:egfp zebrafish were obtained from Zebrafish International Resource Center (University of Oregon) and maintained under standard breeding conditions at 28°C (Lawson and Weinstein, 2002). Embryos were dechorionated using pronase enzymatic treatment and 6 ng of splice/translation blocking netrin-1, UNC5B and control morpholinos that were previously designed and characterized were injected alone or in combination in the high yolk of one to four-cells-stage embryos. A third splice-blocking morpholino specifically targeting netrin-1a was designed (GeneTools) and injected in embryos at the same dose. Finally, 3 ng of a translation-blocking morpholino targeting zebrafish ortholog of DAP kinase (GeneTools) was injected, leading to 35 % decrease of DAP kinase mRNA expression in knocked-down embryos compared to control. Sequences of morpholinos are available upon request. Embryos were raised in E3 medium supplemented with 0.003 % phenylthiourea to inhibit pigment formation. Rescue of phenotype induced by netrin-1a silencing was tested by adding 40 nM BAF in the E3 medium 4 hours after morpholinos injection. For TUNEL staining, embryos were fixed at 48 hpf in 4 % PFA overnight at 4°C. They were then rinsed 10 minutes in PBS-Tween 0.1 % (PBST) and progressively dehydrated in successive methanol baths (25, 50, 75 and 100 %). After rehydratation (75, 50 and 25 % methanol series), embryos were rinsed in PBST, treated 5 minutes with 10 µg/ml proteinase K (Roche) in PBS, rinsed again in PBST and incubated in PBS-BSA 1 % for 30 minutes. TUNEL labeling was achieved as previously stated above and revealed using Cy3-coupled-streptavidine (1/400; Jackson Immunoresearch). Caspase-3 activity assay was performed as described above on whole embryos at 24 h development. For time-lapse experiments, zebrafish embryos were embedded in a 3 % methylcellulose solution with 0.016 % tricaine to inhibit movements (Isogai et al., Development and Disease, 2003). Transmission videomicroscopic imaging was performed using Timelapse Axiovert100M (PLATIM, ENS Lyon).

### EXAMPLES

The inventors have investigated here whether these two apparently opposite observations could be explained by the ability of netrin-1 to block apoptosis induced by its unbound UNC5B receptor and thus promote survival of endothelial cells during angiogenesis. Indeed, netrin-1 prevents epithelial cell death by acting as a ligand for the so-called dependence receptors DCC and UNC5A-D, which share the functional property to be active both in presence and in absence of their ligand: whereas ligand binding triggers positive signaling, dependence receptors induce another signaling cascade leading to cell death when disengaged from their trophic ligand (Llambi et al., 2001; Mehlen et al., 1998; Tanikawa et al., 2003) (Bredesen et al., 2005; Mehlen and Bredesen, 2004). In this scheme, loss of UNC5B -i.e., loss of pro-apoptotic signaling-would indeed be associated with an increased number of blood vessels (Lu et al., 2004). On the contrary, loss of netrin-1 -i.e., increase of pro-apoptotic signaling- is expected to be associated with vessels disappearance (Wilson et al., 2006). Such a dynamic balance between endothelial cells survival and apoptosis could help to maintain blood vessels integrity, which is in turn essential for vascular homeostasis, both during vascular development and pathological angiogenesis as previously described (Alavi et al., 2003; Carmeliet, 2005; Duval et al., 2003; Santoro et al., 2007; Winn et al., 2005).

### Example 1: Netrin-1 blocks endothelial cell apoptosis induced by its unbound UNC5B receptor

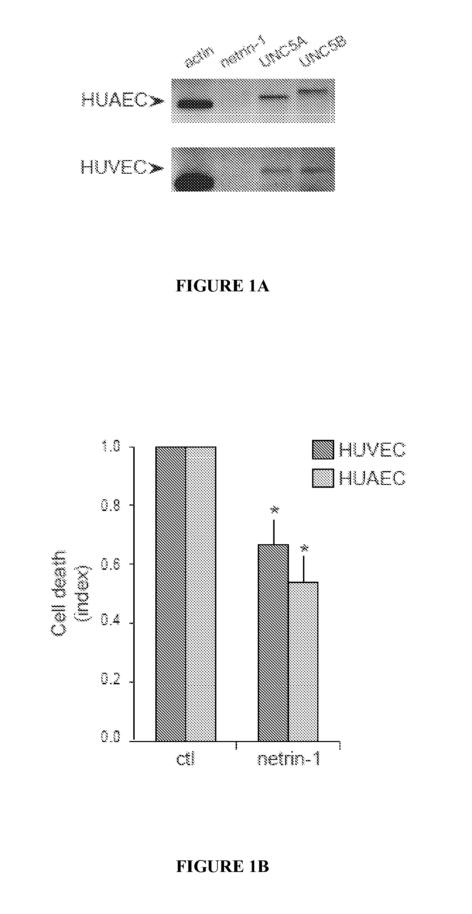

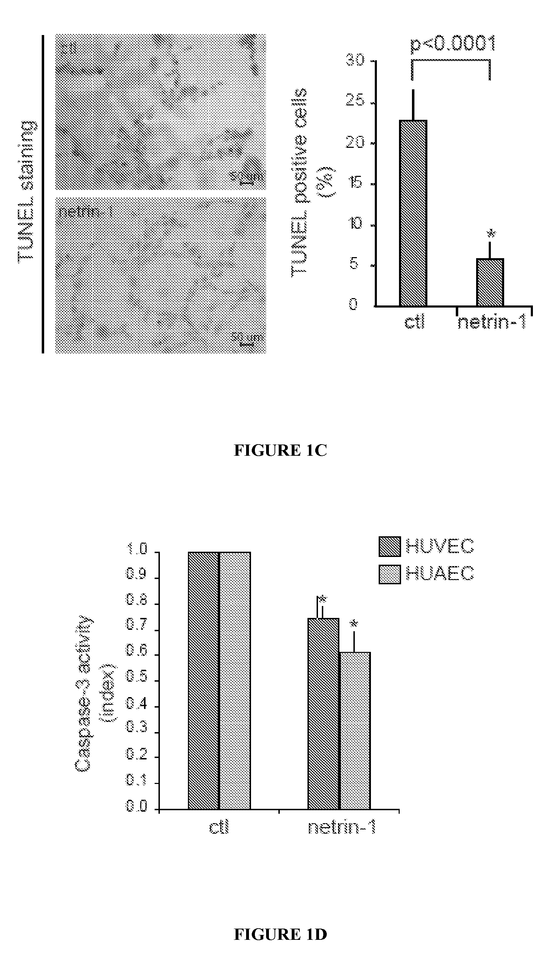

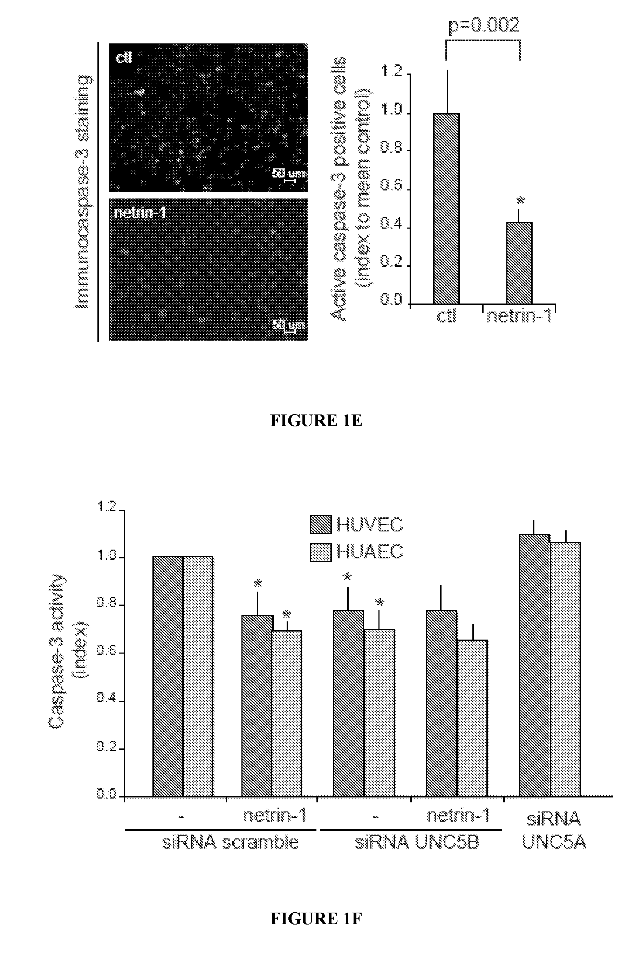

To determine whether netrin-1 blocks apoptosis induced by its unbound UNCB receptor, we first determined which netrin-1 dependence receptors -i.e., DCC, UNC5A, UNC5B, UNC5C and UNC5D- are expressed in human umbilical vein/artery endothelial cells (HUVEC/HUAEC). As revealed by Q-RT-PCR, UNC5A and UNC5B are both expressed in HUVEC and HUAEC (Figure 1A), while we failed to detect UNC5C, UNC5D and DCC (data not shown). Netrin-1 was not expressed in these cells, either at the RNA (Figure 1A) or protein level (Figures 4A-4B). We then investigated whether netrin-1 behaves as a survival factor for endothelial cells by analyzing its ability to block the spontaneous propensity of HUVEC and HUAEC to undergo cell death under serum starvation. Netrin was used at 50 ng/ml in all in vitro experiments, since this dose was previously described to be physiological and functional ((Serafini et al., 1996; Serafini et al., 1994) and Figure 4C). As shown in Figure 1B, netrin-1 inhibited both HUVEC and HUAEC cell death (p<0.005). Similar results were obtained for etoposide-induced cell death (Figure 4D). Netrin-1 prevented endothelial cell apoptosis as shown by the decreased number of TUNEL positive cells (Figure 1C) and by the reduced caspase-3 activity (Figures 1D-1E) (up to 30 % for HUVEC and 40 % for HUAEC; all p<0.005). The addition of a recombinant protein corresponding to the extracellular domain of UNC5H2 prevented netrin-1-mediated cell death inhibition, supporting the conclusion that the survival effect is specific for netrin-1 (Figure 4E). Thus, netrin-1 behaves as a survival factor for endothelial cells.

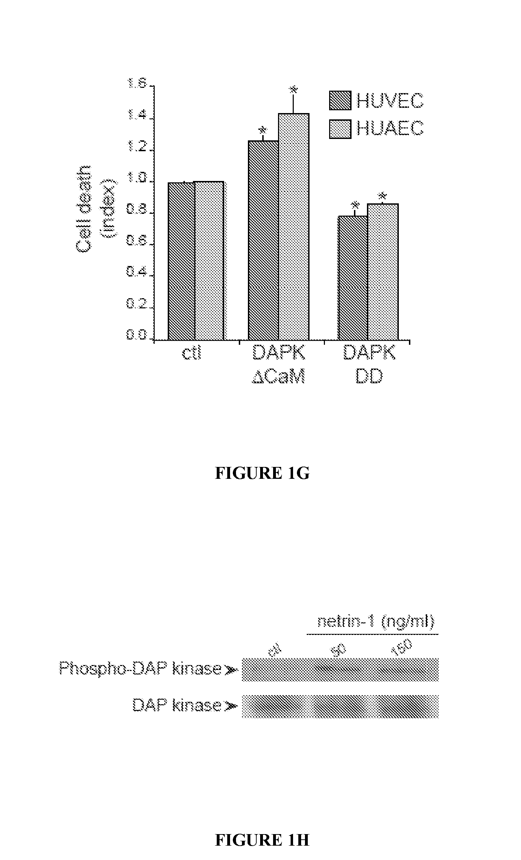

To determine whether UNC5B and/or UNC5A induce apoptosis in endothelial cells, we transiently silenced their expression using a siRNA strategy (Figure 1F and Figures 4F-4G). UNC5B silencing led to a decreased caspase-3 activity, whereas UNC5A siRNA had no effect on caspase-3 activity (Figure 1F; all p<0.05). Moreover, the addition of netrin-1 together with UNC5B silencing was not associated with an additional survival effect (Figure 1F). Thus, the netrin-1 survival effect on endothelial cells depends on UNC5B expression and likely results from an inhibition of apoptosis that would otherwise be triggered by unbound UNC5B. Because UNC5B-induced apoptosis has been shown to be mediated by DAP kinase, we first expressed the constitutively active DAP kinase, DAPK ΔCaM (Shohat et al., 2001) in HUVEC and HUAEC cells. As shown in Figure 1G, expression of the constitutively active form of DAP kinase led to increased cell death (all p<0.0001). We next forced expression of the dominant negative mutant of DAP kinase, DAPK DD (Llambi et al., 2005). Similarly to netrin-1 treatment, DAPK DD prevented cell death induction. Moreover, because UNC5B pro-apoptotic signaling is mediated by the activation of DAP kinase through the inhibition of DAP kinase autophosphorylation (Llambi et al., 2005), we next measured DAP kinase phosphorylation level. As shown in Figure 1H, netrin-1 treatment led to an increase in DAP kinase phosphorylation in HUVEC, presumably by blocking the UNC5B pro-apoptotic signaling cascade. Together with the observed increase in number of blood vessels in UNC5B mutant embryos (Lu et al., 2004), these in vitro data support the view that netrin-1 is capable of blocking UNC5B-induced apoptosis during angiogenesis.

### Example 2: The netrin-1 pro-angiogenic effect is mimicked by caspase inhibition and by silencing of UNC5B

We next challenged the notion that the pro-angiogenic activity of netrin-1 is related to its ability to promote cell survival, using ex vivo models of neoangiogenesis. We first used a mouse aortic ring assay and assessed the expression of UNC5H2, the murine ortholog of UNC5B, in the aortic explants (Figure 5A). Netrin-1 was not found to be expressed in the explants (data not shown). Similarly to what has been observed with VEGF, netrin-1 treatment enhanced capillary sprouting from matrigel-embedded aortic rings, in comparison to control conditions (Figures 2A-2B; p<0.001), supporting a pro-angiogenic activity of netrin-1. Interestingly, caspase inhibitors z-VAD-fink and BAF also induced neocapillary tube formation, mimicking the effect of netrin-1 (Figures 2A-2B; p<0.002). Identical results were obtained in the chick-chorioallantoic membrane (CAM) assay (Figures 2C-2D and Figure 5B for expression analysis). Indeed, we observed a significant increase in neovessel formation in the vicinity of methylcellulose discs soaked either with VEGF, netrin-1, z-VAD-fmk or BAF (Figures 2C-2D; p<0.02). Thus, in these ex-vivo physiological models, inhibition of apoptosis appears to be sufficient to mimic the effects of netrin-1. Moreover, UNC5B silencing by siRNA in CAM (Figure 5C) led to an increase in neovessel ramifications, demonstrating that netrin-1 and its receptor UNC5B act in an antagonistic manner during the angiogenic process (Figures 2E-2F; p=0.009).

### Example 3: Netrin-1a silencing during zebrafish development leads to vascular defects that are rescued by caspase inhibition and by UNC5B/DAPkinase silencing

To further explore in vivo the cause-and-effect relationship between the pro-angiogenic and the anti-apoptotic activities of netrin-1, we investigated whether anti-apoptotic treatment could rescue vessel defects induced by netrin-1 disruption during zebrafish development. Two netrin-1 genes orthologs have been characterized in zebrafish, netrin-1a and netrin-1b (Lauderdale et al., 1997; Strahle et al., 1997). While netrin-1b silencing had no obvious effect on vessel development (Figure 6A), both Eichman's group and Li's group have shown, using respectively different netrin-1a morpholinos, that silencing of netrin-1a is associated with vessels development defects (Lu et al., 2004; Wilson et al., 2006). Netrin-1a silencing using a splice-blocking morpholino (spl-netrin-1a) has been shown to prevent the formation of parachordal vessels (PAVs) (Wilson et al., 2006). Using fli:egfp transgenic zebrafish -a transgenic model targeting the expression of GFP specifically in blood vessels (Lawson and Weinstein, 2002)- we indeed observed at 50-54 hours post-fertilization (hpf) that 57.3 % of the embryos injected with such spl-netrin-1a morpholinos lacked PAVs (n = 75), while the development of intersegmental vessels (ISVs) and dorsal longitudinal anastomotic vessels (DLAVs) was normal (Figure 3A). Treatment of the spl-netrin-1a injected embryos with the pan-caspase inhibitor BAF successfully reversed this phenotype, since PAV formation occurred in 92.6 % of treated embryos (n = 54) compared to 42.7 % in untreated ones (Figure 3A; p<0.0001). As described previously (Wilson et al., 2006), we observed that netrin-1a silencing using a translation-blocking morpholino (tsl-netrin-1a, Figure 6B), as well as a third netrin-1a blocking morpholino (Figure 6C), led to the disorganization of ISVs, to the absence of the dorsal anastomotic vessel (DLAV) (Figure 3B), and also to developmental defects. Time-lapse analysis of developing ISVs confirmed the absence or premature arrest of ISVs (See Example 4). Similar defects were previously described in zebrafish embryos knocked down for the small inhibitor of apoptosis protein survivin (Ma et al., 2007). Moreover, an increase in caspase-3 activity was observed in netrin-1a knock-down embryos (Figure 6D). Treatment of these embryos with BAF was sufficient to rescue ISV formation, since 54.5 % of treated embryos displayed at least partial ISVs compared to 23.0 % in controls (Figure 3B; p<0.001). In agreement with the dependence receptor notion, simultaneous injection of UNC5B morpholinos (Figure 6B) was also able to correct the defects induced by tsl-netrin-1a morpholino injection in 79.6 % of the embryos (n = 54) (Figure 3B; p<0.0001). Similar rescue was obtained by co-injecting tsl-netrin-1a blocking morpholinos and morpholinos targeting DAP kinase (Figure 3B; p<0.0001).

BAF treatment or UNC5B morpholino injection both rescued the increase of cell death observed in embryos after netrin-1a silencing, as measured by the TUNEL immunostaining assay and a caspase-3 activity assay (Figure 3C), hence providing further support for the idea that the endothelial cell death inhibited by netrin-1a is specifically mediated by UNC5B during vessel development.

### Example 4: Time-lapse analysis of netrin-1a morpholino effect on trunk vascular development in fli-EGFP zebrafish embryos

Timelapse images of vessel dynamics in fli-EGFP transgenic zebrafish can be obtained. Initial emergence of intersegmental vessels (ISVs) can be thus shown with time points collected with 3 minutes between time points.

These timelapse images (data no shown) that ISVs and dorsal longitudinal anastomotic vessels (DLAVs) formation occur normally in fli-EGFP zebrafish embryos injected with control morpholinos. In fli-EGFP zebrafish embryos injected with translation-blocking netrin-1a morpholinos, it is demonstrated that ISVs fail to form or present premature growth arrest and DLAVs is absent.

The inventors have shown here that netrin-1 controls the survival of endothelial cells and promotes angiogenesis, at least in part by blocking apoptosis induced by its unbound UNC5B receptor. Deregulation of this function could explain the modulation of blood vessel formation that we and others have observed (Lu et al., 2004; Wilson et al., 2006). This is in agreement with growing lines of evidence describing the importance of cell death regulation during developmental and pathological angiogenesis (Birdsey et al., 2008; O'Connor et al., 2000; Santoro et al., 2007).

Netrin-1 dependence receptors were recently proposed to act as tumor suppressors by inducing apoptosis of epithelial cells that would otherwise develop in settings of trophic ligand unavailability (Grady, 2007; Mehlen and Puisieux, 2006). Loss of the pro-apoptotic activity of its dependence receptors therefore represents a selective advantage for a tumor cell, as exemplified by the frequent loss of DCC and UNC5C expression in colorectal cancer (Bernet et al., 2007; Mazelin et al., 2004). Complementarily, gain of netrin-1 expression represents a similar selective advantage for tumor cells, and this phenomenon is indeed observed in some types of aggressive cancers, such as metastatic breast cancers and lung cancer (Delloye et al., 2008; Fitamant et al., 2008). In light of the results presented here, we propose that this autocrine netrin-1 gain of expression in tumor epithelial cells may have two additive effects. First, as reported, it confers a selective advantage for epithelial tumor cells by inhibiting dependence receptor-induced cell death (Fitamant et al., 2008). Second, it could also potentially favor blood vessel maintenance and/or development, and consequently promote cancer progression. Therefore, an anti-cancer approach based on disruption of netrin-1 function, by titration or inhibition of the interaction with its receptors, should eradicate not only tumor epithelial cells but also tumor angiogenic vessels, thus further strengthening the position of netrin-1 as a critical target in cancer.

### REFERENCES

Alavi et al. (2003). Science 301, 94-96.
Bernet et al. (2007). Gastroenterology 133, 1840-1848.
Birdsey et al. (2008). Blood 111, 3498.
Bredesen et al. (2005). Cell Death Differ 12, 1031-1043.
Carmeliet, P. (2005). Nature 438, 932-936.
Cirulli et al. (2007). Nat Rev Mol Cell Biol 8, 296-306.
Delloye et al. (2008). J Natl Cancer Ins in press.
Duval et al. (2003). Angiogenesis 6, 171-183.
Fitamant et al. (2008). Proc Natl Acad Sci USA, 105, 4850-4855.
Grady, W.M. (2007). Gastroenterology 133, 2045-2049.
Lauderdale et al. (1997). Mol Cell Neurosci 9, 293-313.
Lawson et al. (2002). Dev Biol 248, 307-318.
Liu et al. (2004). Curr Biol 14, 897-905.
Llambi et al. (2001). Embo J 20, 2715-2722.
Llambi et al. (2005). Embo J 24, 1192-1201.
Lu et al. (2004). Nature 432, 179-186.
Ma et al. (2007). BMC Dev Biol 7, 50.
Mazelin et al. (2004). Nature 431, 80-84.
Mehlen et al. (2004). Apoptosis 9, 37-49.
Mehlen et al. (2006). Nat Rev Cancer 6, 449-458.
Mehlen et al. (1998). Nature 395, 801-804.
Nicosia et al. (1990). Lab Invest 63, 115-122.
O'Connor et al. (2000). Am J Pathol 156, 393-398.
Santoro et al. (2007). Nat Genet 39, 1397-1402.
Serafini et al. (1996). Cell 87, 1001-1014.
Serafini et al. (1994). Cell 78, 409-424.
Shohat et al. (2001). J Biol Chem 276, 47460-47467.
Strahle et al. (1997). Mech Dev 62, 147-160.
Tanikawa et al. (2003). Nat Cell Biol 5, 216-223.
Tauszig-Delamasure et al. (2007). Proc Natl Acad Sci USA, 104, 13361-13366.
Wilson et al. (2006). Science 313, 640.
Winn et al. (2005). J Thromb Haemost 3, 1815-1824.

## Claims

1. Method for selecting a compound capable to induce the death of endothelial cells, wherein said method comprises the following steps of:
a) having a medium containing netrin-1, or a fragment thereof, and endothelial cells;
b) contacting said medium with the compound to be tested;
c) measuring the death of said endothelial cells; and
d) selecting said compound if the measuring in step c) demonstrates a significantly induction of the death of the endothelial cells in presence of said compound.

2. Method for selecting a compound capable to induce the death of endothelial cells, wherein said method comprises the following steps of:
a) having a medium containing endothelial cells which express at least one netrin-1 receptor;
b) contacting said medium with the compound to be tested;
c) measuring the ability of said compound to be tested to promote the pro-apoptotic activity of this netrin-1 receptor; and
d) selecting said compound as an inducer of the death of endothelial cells, if the measuring in step c) demonstrates that said compound promotes significantly the pro-apoptotic activity of this netrin-1 receptor, preferably in presence further of netrin-1.

3. Method for selecting a compound capable to induce the death of endothelial cells according to claim 2, wherein:
- the measure in step c) consists to measure the ability of the compound to be tested to induce the activation of the kinase activity of the death-associated protein kinase (DAPK); and
d) selecting said compound as an inducer of the death of endothelial cells, if the measuring in step c) demonstrates a significantly activation of the kinase activity of the death-associated protein kinase (DAPK) in presence of said compound, preferably in presence further of netrin-1.

4. Method for selecting a compound capable to induce the death of endothelial cells according to claim 2, wherein:
- the measure in step c) consists to measure the ability of the compound to be tested to inhibit the DAPK phosphorylation; and
d) selecting said compound as an inducer of the death of endothelial cells, if the measuring in step c) demonstrates a significantly inhibition of the DAPK phosphorylation in presence of said compound, preferably in presence further of netrin-1.

5. Method for selecting a compound capable to induce the death of endothelial cells, wherein said method comprises the following steps of:
a) having a medium containing endothelial cells which express at least one netrin-1 receptor and wherein said netrin-1 receptor intracellular domain is able to dimerize or multimerize in presence of netrin-1;
b) contacting said medium with the compound to be tested, the medium further containing netrin-1, or a fragment thereof able to interact with the extracellular domain of the netrin-1 receptor;
c) determining whether the dimerization or multimerization of said netrin-1 receptor intracellular domain is inhibited in presence of said compound to be tested; and
d) selecting said compound as an inducer of the death of endothelial cells, if the determination in step c) demonstrates a significantly inhibition of the dimerization or multimerization of the intracellular domain of said netrin-1 receptor of said endothelial cells.

6. The method according to one of claims 1 to 5, for selecting a compound capable to induce the death of the blood vessels or neovessels.

7. The method according to one of claims 1 to 6, wherein at step a) said endothelial cells are endothelial cells which express at least a netrin-1 receptor selected from the group of DCC, UNC5A, UNC5B, UNC5C and UNC5E, neogenin and the adenosine A2b, preferably UNC5B.

8. The method according to claim 7, wherein at step a) said endothelial cells are endothelial cells which express the netrin-1 receptor UNC5B.

9. The method according to one of claims 1 to 8, wherein at step a) said endothelial cells are endothelial cells which are selected from the group consisting of human umbilical vein endothelial cells (HUVEC) and human arterial endothelial cells (HUAEC).

10. A compound selected from the group consisting of:
- a compound comprising an extracellular domain of netrin-1 receptor or fragment thereof able to specifically inhibit the interaction between the netrin-1 and said netrin-1 receptor;
- a monoclonal or polyclonal antibody directed specifically against netrin-1 or netrin-1 receptor, particularly directed to the extracellular domain of said netrin-1 receptor or to the netrin-1 fragment able to interact with the extracellular domain of said netrin-1 receptor; and
- a compound capable of inhibiting the netrin-1 expression selected from the group consisting of antisense and iRNA (interfering RNA) oligonucleotides specific of the nucleic acid encoding netrin-1 protein,
as an inducer of the death of endothelial cells.

11. A compound according to claim 10, for its use for inducing the death of blood vessels or neovessels.

12. A compound according to claim 10 or 11, for its use for inducing the death of blood vessels or neovessels of tumors.

13. A compound according to claim 12, wherein the tumor cells of said tumor express or overexpress netrin-1.

14. Kit for the selection of a compound capable to induce the death of endothelial cells, preferably endothelial cells from vessels or neovessels, wherein said kit comprises:
- a netrin-1 protein or a fragment thereof able to specifically interact with netrin-1 receptor protein to form a binding pair; and
- endothelial cells which express a netrin-1 receptor, preferably selected from the group consisting of DCC, UNC5A, UNC5B, UNC5C and UNC5D, neogenin and the adenosine A2b, more preferably the Netrin-1 receptor UNC5B.

15. Kit according to claim 14, wherein said endothelial cells are selected from the group of HUVEC and HUAEC cells.
